# EUROPEAN PATENT SPECIFICATION

(11) **EP 1 541 192 B1**
(45) Date of publication and mention of the grant of the patent: **10.12.2008**
(21) Application number: 03784214.3
(22) Date of filing: 01.08.2003
(51) Int. Cl.: A61N 1/36, A61N 1/362

(54) **TWO-PHASE CURRENT VENTRICULAR ELECTRICAL STIMULATOR FOR HEART FAILURE AND STIMULATION METHOD**
VENTRIKULÄRER ELEKTRISCHER ZWEIPHASEN-STROM-STIMULATOR GEGEN HERZINSUFFIZIENZ UND STIMULATIONSVERFAHREN
STIMULATEUR ELECTRIQUE VENTRICULAIRE A COURANT BIPHASIQUE POUR L'INSUFFISANCE CARDIAQUE ET PROCEDE DE STIMULATION

(30) Priority: 07.08.2002 ES 200201876
(43) Date of publication of application: 15.06.2005
(73) Proprietor: UNIVERSIDADE DE SANTIAGO DE COMPOSTELA, 15782 Santiago de Compostela (ES)
(72) Inventor: GARCIA-BENGOCHEA GONZALEZ-MORO, José, Benito, 15782 Santiago de Compostela (ES)
(74) Representative: Carvajal y Urquijo, Isabel
(86) International application number: PCT/ES2003/000402
(87) International publication number: WO 2004/014480

(56) References cited:
- CN-A- 1 336 240
- US-A- 4 402 322
- US-B1- 6 341 235

## Description

### Field of the Invention

The present invention belongs to the field of single ventricular chamber or dual atrial-ventricular chamber electrical heart stimulators or pacemakers, acting by means of the application of a biphasic shock current, and particularly refers to a pulse generator of biphasic current transmitted through electrodes to the ventricles for treating heart failure caused by a loss of ventricular synchronism.

### Background of the Invention

Heart failure due to severe ventricular dysfunction currently represents one of the greatest medical problems in the Western world, from the medical point of view as well as from the social and economic perspectives.

According to estimates of the AHA (American Heart Association), five million people suffered from this disease in 1999 in the United States, and in that same year, the United States Health Department considered that approximately 160 million people worldwide would be in need of sophisticated treatment due to this type of heart failure.

On the other hand, 70% of those affected are over the age of 65 years, and hospitalization cost in the United States due to heart failures exceeds 3.8 billion dollars with an increase of approximately 445,000 patients per year also being verified.

These epidemiological data, to which constant aging of the population must be added, provide a clear idea of the magnitude implied by the problem of heart failure treatment, becoming critical when it reaches refractory to maximum, optimal pharmacological treatment.

In refractory heart failure, an event called "desynchronization" of contractile activity of the ventricles occurs, severely affecting the pumping behavior of said chambers (Herman MW et al., in "Localized Disorders in Myocardial Contraction: Asynergy and Its Role in Congestive Heart Failure", N. Engl. J. Med., 1967, 277, pp. 222-232).

In dilated cardiomyopathies (contractile dysfunction due to dilatation of the ventricular cavities), it has been observed that structural loss of collagen matrix affects intraventricular electrical conduction and the coordinated contractile response of the various segments of the ventricle. The result is the loss of contractile efficiency in one ventricle, the function of which is already altered.

Another cause of contractile asynergy is the presence of blocks in the intraventricular electrical conduction tissue and, specifically, the Bundle of His block, resulting in a delay in the arrival of the electrical stimulus to the ventricle and affecting up to 30% of dilated cardiomyopathies.

Lastly, another form of ventricular asynergy is found in ventricles suffering from several types of regional ischemia, i.e. lack of blood supply, due to the total or partial obstruction of the coronary arteries; for this reason, a lack of correspondence or a disproportion between the supply and demand is created. Certain segments of these ventricles, with an earlier activation, will contract with a minimum volume load, but the shortening of these fibers does not translate into the creation of significant pressure since other segments are still inactive or are activated later and, furthermore, the latter need a large load (pressure) to respond and contract.

All these abnormal mechanisms finally lead to transferring the ejection from one part of the ventricular chamber to another part, without the amount of blood being ejected into systemic circulation through the aortic valve while it is open.

In general terms, from the hemodynamic point of view, the combination of processes generate an extension of the ventricular pre-ejection time, shortening of the ejection and relaxation times, and subsequent decrease of the ventricular ejection fraction, in addition to causing an increase of the mitral valvular insufficiency, almost always existing already due to ventricular dilatation.

In 1966, Schlant was the first to disclose the systolic function improvement resulting from a coordinated ventricular segment activation. This mechanism was called "idioventricular kick" (Circulation, 1966, 23, (Suppl. III): 209, Abstract).

Research carried out in 1982 revealed which were the most suitable areas of the heart for chronic electrical pacing, i.e. for implanting permanent pacemaker electrodes, by means of epimyocardial cartography, it being found that not all areas are suitable and that the right ventricle, in its diaphragmatic surface as well as in its anterior segment, needed a greater electrical stimulus than the left ventricle; i.e. the electrical pacing threshold was higher and, furthermore, right pacing generated paradoxical septal motion in the left ventricle. On the other hand, it was also noticeable that left pacing through a thoracotomy provided an improvement in some patients suffering heart failure, although it could not be clarified in which ones.

In more recent hemodynamic studies, by means of the implantation of temporary electrodes in both the right and left ventricles in patients with a low cardiac output and an ejection fraction of less than 35%, and subjected to extracorporeal circulation, it could be noticed that right ventricular pacing only serves to modify the heart rate, which concludes in a routine matter in conventional pacemaker implantation, and that only electrical pacing of the left ventricle, or of both ventricles simultaneously, is able, in certain patients presenting ventricular desynchronization, to improve ejection capacity, mainly at the expense of reversing the paradoxical motion of the ventricular septum, a very frequent phenomenon in post-heart operation patients.

More recently in 1994, the concept of "ventricular resynchronization" was introduced in clinical practice by Bakker et al. (Pacing Clin. Electrophysiol., 1994; 17:280, Abstract) and later, with more cases, by Cazeau et al. (Pacing Clin. Electrophysiol., 1996; 19:1748-1757), and is derived from the normal use of dual-chamber or atrial-ventricular pacemakers, also called DDD pacemakers in international code. These authors showed that implanting electrodes in both ventricles and stimulating the atrium and the two ventricles, or only the left ventricle, in a synchronized manner in patients with left of the Bundle branch block, an increase of the ejection fraction was found, although they did admit that, given the variability of results, better patient screening was necessary due to the different heart failure etiologies.

At the same time, other researchers (Aurichio A. "Ventricular resynchronization: basis, technical problems and preliminary results", Abstract. The Euro contribution to arrhythmology, 28 February-March 2002, Madrid) have also clearly shown the importance of establishing a correct atrial-ventricular delay in ventricular resynchronization by means of Eco-Doppler evaluation, such that the emptying-filling phase of the chambers is maximally taken advantage of (Kass et al., "Improved Left Ventricular Mechanics from Acute VDD Pacing in Patients with Dilated Myocardiopathy and Ventricular Conduction Delay", Circulation 1999, 30 March; 99 (12), pp. 1567-1573).

Lastly, the multicenter European study, "The InSync Italian Registry (2000) (European Heart Journal (Suppl) J22-J30") established "resynchronization" as a new alternative in the treatment of ventricular failure, pointing out, however, that the type of pacing which must be carried out, or if it is to be carried out in both ventricles or only in the left ventricle, and in which area of the latter, is not completely clarified. These aspects are being clarified by means of the use of tissue Doppler, Carto-Graph and nuclear magnetic resonance, which allow visualizing the areas which are activated later.

In 1999, and based on the researcher's own experience acquired in heart surgery, implanting temporary electrodes in ventricles for electrical pacing of post-heart operation patients with extracorporeal circulation and with an ejection fraction of less than 35%, it could be shown, by means of determinations by thermodilution with a Swan-Ganz catheter and by means of Doppler echocardiography, that simultaneous biventricular or isolated left ventricular pacing was able to improve left ventricular function, i.e. the heart output in liters/minute, and that, in some cases, at the expense of the reversal of the paradoxical septal motion usually generated after heart surgery achiving a simultaneously increased of the ejection fraction and other hemodynamic parameters.

Therefore, heart failure patients with desynchronization and bradycardia began to be treated by means of implanting electrodes in the right atrium (if they showed normal, i.e. sinus, rhythm) and in the two ventricles or only in the left ventricle. The evolutions, followed by means of Doppler echocardiography, showed different degrees of improvement of ventricular function and of functional ability in spite of the heterogeneity of the treated population.

Another important aspect, a product of the observations of the inventor of the present application, is that in order to obtain resynchronization in a more effective and constant manner, it is advisable to pace the segments of the ventricle which are later activated, as is visualized by means of the tissue Doppler, and, in the cases with intraventricular conduction disorder, this segment is usually the free wall of the left ventricle accompanying abnormal motion of the intraventricular septum, whether it is akinetic or paradoxical motion. However, it was impossible to show in an overall manner if the stimulation was better in the left ventricle alone or in both ventricles, since complete resynchronization was not always achieved.

It could later be shown, by means of echocardiography, that anomalous septal motion, very common after heart surgery and which generates ventricular asynchronism, with the subsequent adverse hemodynamic effect, is reversible as a result of ventricular pacing with coordinated biphasic current. This means that, since a large majority of patients with cardiomyopathy present inter or intraventricular conduction disorders, it is impossible to predict, for now, where the shock of the two-pulse current is going to occur, therefore, according to echocardiographic control, it is necessary to move up or delay one of the two electrical pulses.

This positive effect has been corroborated in later phases, very satisfying pacing thresholds also being obtained, and it being shown, by means of measurement of the intramyocardial tissue oxygen pressure, that there are no significant alterations with regard to pacing with single-phase current.

Until now, the type of conventional pacing used for achieving ventricular resynchronization with single-phase current consists of the electrical pulse closing the circuit between two electrodes, one located in each ventricle, or separately in the left ventricle. In other words, two unipolar electrodes are implanted, one in each ventricle, linked to the current generator (pacemaker) by means of a "Y" connector, and the generator programmed in bipolar version. Thus, all that is achieved is being able to choose the direction of the current between the two electrodes, one acting as a positive pole (anode) and the other as a negative pole (cathode). The bipolar programming would consist of this.

Another pacing form would be unipolar pacing, closing the circuit between an electrode and the generator casing, and the tip of the electrode always acting as a cathode and the generator casing as an anode. The alternative for resynchronization in left ventricular pacing requires the left ventricular electrode to be the cathode.

If a ventricular or single-chamber (VVI), i.e. with a single output, generator is used, a "Y" connector must be used to receive the two electrodes, it being necessary to know where the negative pacing, i.e. the proximal tip of the connector, is located, because, in this manner, when choosing between the electrode/electrode (bipolar) variant and the electrode/casing (unipolar) variant, it will determine which ventricle the stimulus is to be generated in. It is evident that it will be necessary for the stimulus triggering to occur in the left ventricle in order to obtain resynchronization.

Another resynchronization possibility which has been used consists in triggering the stimulus in the two ventricles simultaneously or with a small delay. This is achieved by means of a dual-chamber (DDD) generator provided with an atrial-ventricular (A-V) delay equal to zero, implanting two electrodes, one in each ventricle. Thus, two stimuli will be simultaneously generated, the two negative poles being the active terminal parts of the electrodes and the circuit being closed with the generator casing.

In this type of pacing, it is necessary to predetermine which is the ventricle to be contracted in the first place, at a given time, since modifying the A-V delay will achieve that the ventricle connected to the atrial output of the generator could receive the stimulus several milliseconds before the other ventricle, or at the same time if the delay is programmed at zero.

The-drawback of this design is based on the fact that these dual-chamber (DDD) generators with A-V delay equal to zero have not been designed for biventricular pacing, but rather for atrial-ventricular pacing, and therefore provide a rather unorthodox pacing for conventional follow-up, naturally not familiar with this technique.

In the cases in which the patients maintains his/her physiological atrial rhythm, i.e. sinus rhythm, it is mandatory to preserve A-V synchronism, in other words, to stimulate both chambers in a synchronous manner, i.e. DDD.

There is also a type of generator called three-chamber in which, in the first place, an output is provided for the atrial electrode, which carries out the perception and pacing functions and, secondly, is provided with two outputs for ventricular pacing, one for the right ventricular electrode and another one for the left, one acting as an anode and the other as a cathode, respectively. This generator is the most expensive of the currently commercially available generators, but it maintains the same system already disclosed.

In many cases, if it is accepted that to achieve resynchronization it is not necessary to carry out biventricular pacing and only left ventricular pacing is necessary, then an atrial and another ventricular output can be provided, adding a "Y" connector and programming the ventricular chamber to bipolar, thereby achieving closing the circuit between both electrodes, of which electrodes one acts as a cathode and the other as an anode. Thus, A-V synchronism is preserved, and ventricular resynchronization is achieved.

However, it must be emphasized that it is always stated that (except in the case of DDD with variable A-V delay, possibly reaching 0 ms) starting from one stimulus, the ventricular anode can be arranged in the desired area, the same occurring with the cathode and closing the circuit between them. In other words, orienting the direction of the current as desired is thus achieved, but nothing more.

In the case of the variable A-V delay DDD, the pathway consists in providing the two electrodes in the desired position, the circuit being closed with the generator casing. A pacer output circuitry for providing biphasic stimulation pulses to heart tissue at a voltage twice the supply voltage is disclosed in document US-A-4 402 322.

### SUMMARY OF THE INVENTION

The present invention is a stimulator as defined in claim 1.

According to a first preferred embodiment of the invention, it starts from a generated positive pulse, which is made to pass through a converter dividing it, a positive polarity pulse and another negative polarity pulse simultaneously being provided, each one of which is transmitted to a ventricular output ring of the generator by means of an output conductor, and each one of which will close the circuit with the generator casing.

A second embodiment of the invention, causing the same effect, consists in arranging a bifurcated output, i.e. with two connections to the generator for each one of the ventricular electrodes, in the pulse generator, it thereby being possible to discard the "Y" connector and there being no device between the electrodes and the generator.

According to a third embodiment of the present invention, an additional output is provided in a generator of this type, whether it is provided with a coaxial output (first embodiment) or if the output is bifurcated (second embodiment), for standard atrial pacing and perception, as a result of which atrial-ventricular synchronism is respected, and a three-chamber pulse generator, but with unipolar ventricular biphasic pacing, is arranged.

### BRIEF DESCRIPTION OF THE DRAWINGS

In the drawings, in the figures of which similar reference numbers designate similar elements,
figure 1 shows a block diagram illustrating a first embodiment of the pacing system according to the present invention;
figure 2 shows a block diagram illustrating a second embodiment of the pacing system of the invention; and
figure 3 and 4 show block diagrams corresponding to the pacing system according to the invention represented in figures 1 and 2, respectively, incorporating an additional variant.

### DETAILED DESCRIPTION OF THE PREFERRED EMBODIMENTS

The present invention is based on the division and transformation of a positive or negative pulse which, when making it go through a signal inverter circuit, achieves changing its polarity.

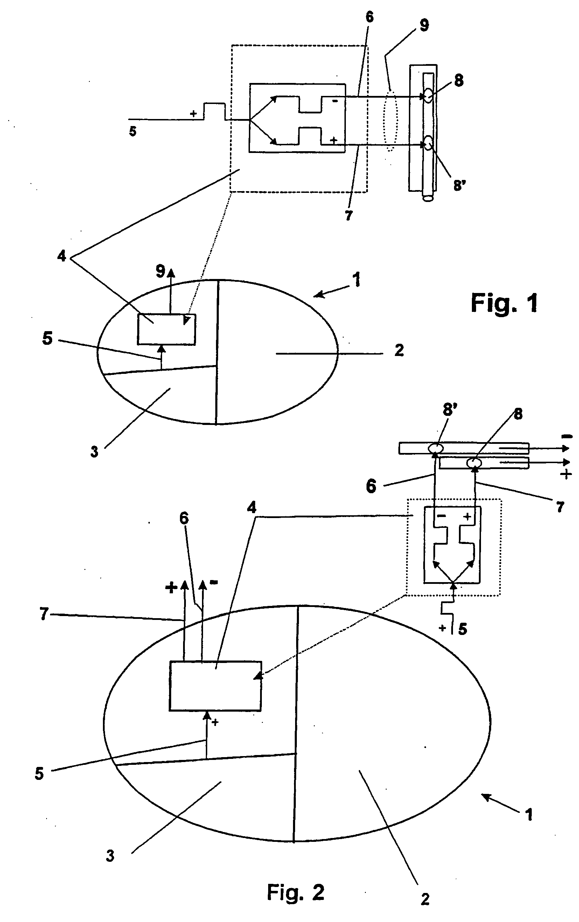

Figure 1 shows a first preferred embodiment of the invention and, in it, an electrical heart stimulator, or pacemaker, is generally designated with 1, schematically constituted of a battery 2 and a VVI pulse generation circuitry 3 provided with the corresponding programming and diagnosing parameters.

A signal- inverter circuit is represented with 4 (see the detail of said circuit enclosed in a dashed line box) intended to receive a pulse through 5, for example, a positive pulse, and to send, through 6 and 7, respective opposing polarity pulses applied directly to the two positive and negative ventricular electrodes 8 and 8', respectively, coaxially arranged 9, therefore it will be provided with a positive stimulus in one electrode and a negative stimulus in the other electrode, therefore, when the simultaneous or variable triggering of both stimuli occurs, a positive and negative current shock, rather than a circuit closure, will be obtained. This biphasic biventricular or univentricular (the two electrodes in the same ventricle) pacing would therefore have a coaxial output.

Figure 2 shows a second preferred embodiment of the invention, consisting in providing the inverter circuit 4 with independent outputs 6, 7, i.e. with respective connections to the generator for the positive and negative ventricular electrodes 8 and 8', respectively, therefore being able to discard the "Y" connecter, and no other device existing between the electrodes and the generator.

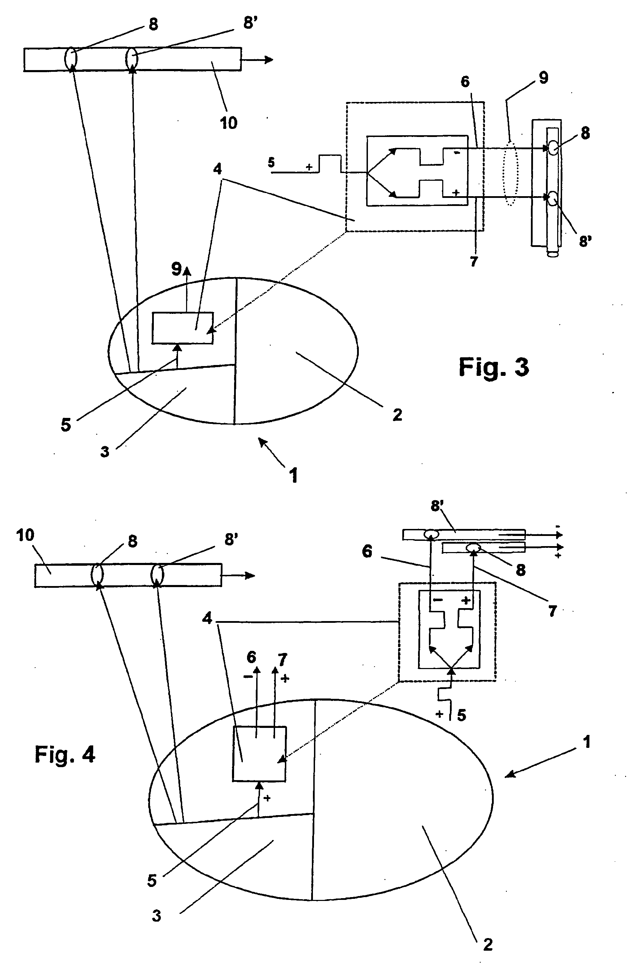

In reference to figures 3 and 4 of the drawings (the inverter circuit 4 is shown in greater detail in figure 3 within a dashed line box), a variant according to the present invention can be seen, incorporated in a generator of this type provided with a coaxial output 9 (see figure 3) and provided with a bifurcated output 6, 7 (see figure 4). According to this variant of the invention, an additional coaxial output 10 is provided for standard atrial pacing and perception, as a result of which atrial-ventricular synchronism is respected, and a three-chamber pulse generator, but with unipolar ventricular biphasic pacing, is provided.

The invention can thus be incorporated in any conventional single-chamber as well as dual-chamber generator (pacemaker) model.

It is also possible to choose between the emission of a conventional biphasic or single-phase pulse, just as it can also be provided with a generator with a coaxial or bifurcated output, it being possible to incorporate polarity to these pulses.

From the medical application point of view, biphasic ventricular pacing as a result of the generated current shock, achieves greater recruitment of myocardial cells between the two electrodes, since it applies to the muscle the entire generated current without dissipation of the latter occurring when the circuit is closed with the generator casing, as occurs in the case of single-phase pacing.

In the case of biphasic pacing, depolarization of the myocardial fiber occurs intercardially by means of a shock of the two stimuli in the area encompassing the muscle mass comprised between the two electrodes with reference to the casing, and not between a point of the muscle with reference to another point or to the casing. On the other hand, given the energy shock generated, the voltage necessary for capture decreases.

Depending on the electrode implantation area and on the distance between the electrodes, it will be achieved that the current shock depolarizes and recruits more or less myocardial mass for the resynchronization and optimization of the ventricular contraction, in turn eliminating the ventricular ectopic foci.

In summary, the standard single-chamber (WIR) or dual-chamber (DDDR) generator, i.e. with response to the frequency, must be provided with a signal converter to transform the ventricular pulse output, either in a bifurcated or coaxial manner, into a biphasic one. Likewise, depending on the type of patient, the atrial-ventricular set could be paced in a synchronized manner by means of modification of the A-V delay, the same which can be done with dual-chamber or DDD generators.

Given the hemodynamic repercussion of resynchronization after heart surgery, both in the surgical act as well as in the recovery rooms, intensive care or coronary intensive care units, the biphasic generator could be implanted for chronic pacing, as described, or for temporary pacing, the converter system for pacing with biphasic current incorporating a conventional external generator.

## Claims

1. A biphasic current electrical ventricular stimulator (1) for heart failure comprising:
a unipolar pulse generator (3) provided with ventricular output electrodes (8, 8') for implantation in the walls of a ventricle,
a direct current feed source (2) for said pulse generator, and
a signal inverter circuit (4) for dividing and transforming the polarity of the output pulses of said pulse generator (3), for generating similar output pulses of reverse polarity, and for simultaneously transmitting the output pulses
and the similar pulses of reverse polarity through at least one output conductor connected to the ventricular electrodes (8, 8').

2. An electrical ventricular stimulator according to claim 1, wherein the transmission of said pulses of opposite polarity from said pulse generator (3) to said ventricular electrodes (8, 8') is carried out by means of a single coaxial conductor.

3. An electrical ventricular stimulator according to claim 1, wherein the transmission of said pulses of opposite polarity from said pulse generator (3) to said ventricular electrodes (8, 8') is carried out by means of independent or bifurcated conductors.

4. An electrical ventricular stimulator according to claim 2, wherein if further comprises a separate output (10) for atrial pacing and perception.

5. An electrical ventricular stimulator according to claim 3, wherein if further comprises a separate output (10) for atrial pacing and perception.

## Patentansprüche

1. Elektrischer ventrikulärer Zweiphasenstromstimulator für Herzinsuffizienz, welcher umfasst:
einen unipolaren Impulsgenerator (3), der mit ventrikulären Ausgangselektroden (8, 8') zur Implantation in den Wänden einer Herzkammer,
eine Gleichstromzuführungsquelle (2) für den genannten Impulsgenerator, und
einen Signalwechselrichterkreis (4) zum Teilen und Umwandeln der Polarität der Ausgangsimpulse des genannten Impulsgenerators (3), zum Erzeugen ähnlicher Ausgangsimpulse umgekehrter Polarität, und zum gleichzeitigen Übertragen der Ausgangsimpulse und der ähnlichen Impulse umgekehrter Polarität durch mindestens einen mit den ventrikulären Elektroden (8, 8') verbundenen Ausgangsleiter.

2. Elektrischer ventrikulärer Stimulator nach Anspruch 1, bei dem die Übertragung der genannten Impulse entgegengesetzter Polarität von dem genannten Impulsgenerator (3) zu den genannten ventrikulären Elektroden (8, 8') durch einen einzelnen Koaxialleiter ausgeführt wird.

3. Elektrischer ventrikulärer Stimulator nach Anspruch 1, bei dem die Übertragung der genannten Impulse entgegengesetzter Polarität von dem genannten Impulsgenerator (3) zu den genannten ventrikulären Elektroden (8, 8') durch unabhängige oder gegabelte Leiter ausgeführt wird.

4. Elektrischer ventrikulärer Stimulator nach Anspruch 2, wobei er desweiteren einen getrennten Ausgang (10) zur Vorhofstimulation und -wahrnehmung umfasst.

5. Elektrischer ventrikulärer Stimulator nach Anspruch 3, wobei er desweiteren einen getrennten Ausgang (10) zur Vorhofstimulation und -wahrnehmung umfasst.

## Revendications

1. Stimulateur ventriculaire électrique de courant biphasé (1) pour insuffisance cardiaque comprenant :
un générateur d'impulsions unipolaires (3) pourvu d'électrodes ventriculaires de sortie (8, 8') pour son implantation dans les parois d'un ventricule,
une source d'alimentation en courant continu (2) pour ledit générateur d'impulsions, et
un circuit inverseur de signaux (4) pour diviser et transformer la polarité des impulsions de sortie dudit générateur d'impulsions (3), pour générer des impulsions de sortie similaires de polarité inverse, et pour transformer simultanément les impulsions de sortie et les impulsions similaires de polarité inverse à travers au moins un conducteur de sortie connecté aux électrodes ventriculaires (8, 8').

2. Stimulateur ventriculaire électrique selon la revendication 1, dans lequel la transmission desdites impulsions de polarité opposée à partir dudit générateur d'impulsions (3) audites électrodes ventriculaires (8, 8') est réalisée au moyen d'un conducteur simple coaxial.

3. Stimulateur ventriculaire électrique selon la revendication 1, dans lequel la transmission desdites impulsions de polarité opposée à partir dudit générateur d'impulsions (3) audites électrodes ventriculaires (8, 8') est réalisée au moyen de conducteurs indépendants ou bifurqués.

4. Stimulateur ventriculaire électrique selon la revendication 2, dans lequel il comprend en outre, une sortie séparée (10) pour perception et stimulation auriculaire.

5. Stimulateur ventriculaire électrique selon la revendication 3, dans lequel il comprend en outre, une sortie séparée (10) pour perception et stimulation auriculaire.
